(19) Europäisches Patentamt
European Patent Office
Office européen des brevets

(11) **EP 3 128 984 B1**

(12) **FASCICULE DE BREVET EUROPEEN**

(45) Date de publication et mention
de la délivrance du brevet:
**14.07.2021 Bulletin 2021/28**

(51) Int Cl.:
*A61F 13/62* (2006.01)   *A44B 18/00* (2006.01)
*B32B 5/02* (2006.01)   *B32B 5/12* (2006.01)
*B32B 5/14* (2006.01)   *B32B 5/26* (2006.01)
*B32B 7/04* (2019.01)   *B32B 27/12* (2006.01)
*D04H 1/08* (2012.01)   *D04H 3/14* (2012.01)
*D04H 11/00* (2006.01)

(21) Numéro de dépôt: **15714221.7**

(22) Date de dépôt: **07.04.2015**

(86) Numéro de dépôt international:
**PCT/EP2015/057440**

(87) Numéro de publication internationale:
**WO 2015/155149 (15.10.2015 Gazette 2015/41)**

(54) **NAPPE OU RUBAN A BOUCLES COMPORTANT DES ZONES A FIXATIONS DIFFERENTIELLES ET COUCHE-CULOTTE COMPORTANT UNE TELLE NAPPE**

SCHLEIFENSCHICHT ODER STREIFEN MIT UNTERSCHIEDLICHEN BEFESTIGUNGSBEREICHEN UND WINDEL MIT SOLCH EINER SCHICHT

LOOPED LAYER OR STRIP INCLUDING DIFFERENTIAL ATTACHMENT AREAS AND NAPPY COMPRISING SUCH A LAYER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.04.2014 FR 1400850**

(43) Date de publication de la demande:
**15.02.2017 Bulletin 2017/07**

(73) Titulaire: **Aplix**
**44850 Le Cellier (FR)**

(72) Inventeur: **MAHE, Anthony Bertrand**
**44860 Pont Saint-Martin (FR)**

(74) Mandataire: **Eidelsberg, Olivier Nathan et al**
**Cabinet Aymard & Coutel**
**22 Avenue de Friedland**
**75008 Paris (FR)**

(56) Documents cités:
**EP-A1- 0 800 808   US-A1- 2004 178 544**
**US-A1- 2009 068 393**

• **None**

**Description**

Domaine technique

La présente invention se rapporte à une nappe ou un ruban à boucles, en forme de stratifié, pour former la partie boucles d'un système auto-agrippant à crochets et boucles, notamment pour des couches-culottes et notamment pour former une bande disposée dans la partie avant de la couche-culotte, au niveau de la ceinture en position centrale, bande classiquement appelée "bande confort" ou "landing zone" en anglais, pour permettre la fermeture de la ceinture de la couche-culotte par l'accroche de crochets issus de pattes disposées dans des parties d'extrémités latérales de la partie arrière de la couche-culotte.

En général, cette nappe comporte d'une part un élément support, classiquement un non-tissé ou un film thermoplastique, sur une surface extérieure de laquelle sont imprimés, par application d'une couche d'encre, des motifs ou dessins, notamment en vue de différencier cette zone appelée "landing zone" (zone d'atterrissage pour les crochets) du reste du vêtement pour indiquer à l'utilisateur la zone fonctionnelle de fermeture, c'est-à-dire la zone dans laquelle les crochets doivent être appliqués pour réaliser l'accrochage, et d'autre part un élément à boucles fixé à l'élément support.

Arrière-plan de l'invention

On connaît déjà dans l'art antérieur le fait d'utiliser un tricot comme élément à boucles. Pour fixer le tricot sur son support, il est nécessaire de réaliser de nombreuses étapes, longues et fastidieuses, qui nécessitent une grande précision afin de ne pas coller les boucles sur le support et annihiler leur pouvoir d'accrochage. Cela ne facilite pas une fabrication à grande échelle dans un domaine où pourtant elle est particulièrement recherchée, à savoir celui des couches-culottes jetables.

C'est pourquoi a été développé le principe consistant à remplacer le tricot à boucles par un non-tissé à boucles.

Cependant, plusieurs critères sont recherchés pour ce non-tissé, à savoir on souhaite que les crochets puissent facilement s'y accrocher et notamment donnent à l'utilisateur l'impression de s'accrocher facilement et de réaliser un accrochage performant et/ou satisfaisant au regard de l'utilisateur, tout en assurant cependant une bonne visibilité du dessin (qui est vu par l'utilisateur à travers l'élément à boucles), ainsi qu'une qualité de toucher doux et agréable. Enfin, on souhaite réduire autant que possible le poids de la bande confort en vue de réduire le coût du produit et limiter son impact sur l'environnement une fois utilisé.

Or, réduire le poids de la bande confort implique notamment que le non-tissé à boucles doit être d'assez faible grammage, ce qui est favorable en terme de visibilité à travers. Cependant, ces deux critères sont certes remplis, mais impliquent alors que le nombre de boucles et notamment le nombre d'accroches potentielles est réduit, de sorte que la résistance à l'ouverture au pelage est moins bonne, et en particulier l'appréciation ressentie par l'utilisateur concernant la qualité de l'accrochage est moins bonne.

On connaît également de US 2004/0178544 un composite comportant une structure polymère élastique qui est soudée dans une étape préalable à un non-tissé suivant un motif de soudure en treillis. Ce composite est destiné à être utilisé dans des couches-culottes pour former une liaison élastique entre des pattes à crochets et la couche-culotte, les crochets, pour fermer la couche, venant sur un élément à boucles disposé sur la partie avant de la ceinture de la couche, le composite élastique assurant le maintien élastique de la fermeture de la couche réalisée par l'action des crochets dans l'élément à boucles, ce dernier étant distinct du composite élastique. Pour donner de l'élasticité au composite, après la soudure du non-tissé à la structure polymère, on étire le composite dans une direction parallèle au plan de la structure, ce qui a pour effet de dessouder le non-tissé de la structure polymère le long des cordons de soudure parallèles à la direction d'étirement, de sorte que l'on obtient un composite final soudé uniquement le long des cordons du treillis perpendiculaires à la direction d'étirement (avec une épaisseur de soudure constante, la soudure au niveau des intersections avec les cordons horizontaux dessoudés par l'étirement ayant été partiellement dessoudé de leur partie correspondant aux cordons de soudure dessoudés parallèles à la direction d'étirement), ce qui donne ainsi de l'élasticité au composite dans la direction d'étirement.

On connaît également de EP 0800808, une couche-culotte ayant une fermeture auto-agrippante à crochets et boucles, l'élément à boucles (bande confort) étant disposé sur la partie avant de la couche au niveau de la ceinture et des pattes à crochets étant issues des bords de la partie arrière de la couche pour venir s'accrocher dans l'élément à boucles pour fermer la couche. Le non-tissé à boucles comporte un mélange de deux types de fibres. Les fibres du premier type sont liées entre elles fortement. Les fibres du deuxième type sont liées entre elles également fortement. Les fibres des deux types sont liées entre elles faiblement. Le réseau des fibres de premier type formant l'élément à boucles est lié au réseau des fibres de deuxième type suivant des points de soudure de force faible mais donc constante, et réciproquement.

## EP 3 128 984 B1

Objet et résumé de l'invention

La présente invention vise à surmonter les inconvénients de l'art antérieur en proposant une nappe ou un ruban à boucles en forme de stratifié, notamment destiné à être utilisé en tant que bande confort dans une couche-culotte, qui, bien que pouvant être de faible grammage et donc d'un coût de production bas et permettant une bonne visibilité du dessin à travers le non-tissé à boucles, permet d'obtenir une bonne tenue des crochets et notamment de donner la sensation à l'utilisateur d'une fermeture robuste.

Suivant l'invention, une nappe ou un ruban à boucles en forme de stratifié comportant un élément support intérieur constitué d'un non-tissé, et une pluralité de fibres et/ou filaments fixés sur la face extérieure de l'élément support de manière à former des boucles pour une coopération avec des crochets, la fixation étant réalisée dans au moins une région de fixation tandis que dans au moins une autre région dite bouclée les filaments et/ou fibres ne sont pas fixés à l'élément support pour ainsi former des boucles, est caractérisé en ce que, dans au moins une zone de la au moins une région de fixation la fixation n'est réalisée que de manière partielle tandis que dans au moins une autre zone de la au moins une région de fixation la fixation est réalisée de manière complète.

Suivant la présente invention, on entend par "fixation réalisée de manière partielle", une fixation, notamment par fusion notamment calandrage, qui est telle que lorsqu'un crochet vient pénétrer dans une ou plusieurs des boucles de l'ensemble de filaments à boucles et exerce une traction de pelage normale, la coopération crochet/boucles entraîne la désolidarisation de la zone de fixation partielle au moins en partie de l'élément support pour ainsi agrandir la ou les boucle(s) avec laquelle ou lesquelles coopère le crochet. Dans le même temps, on entend par "zone de fixation complète" une zone de fixation qui est telle que lorsqu'un crochet pénètre dans une ou plusieurs boucles formées par un ou des filaments ou fibres de l'élément à boucles et exerce une traction de pelage normale, la zone de fixation complète ne se sépare pas de l'élément support.

Suivant l'invention, on entend par traction de pelage "normale" la force de traction résultant de l'action d'une personne de force moyenne qui introduit une patte à crochets de dimension usuelle pour une couche-culotte ou une couche à incontinence pour adultes et qui tire vers elle l'élément à crochets après avoir introduit un crochet dans les boucles de l'élément bande confort pour s'assurer qu'il y a un accrochage réalisé.

En particulier, une force normale est considérée par l'homme de l'art comme étant une force comprise entre 2,2 et 13 Newtons (N) exercée sur une distance de 25,4 mm (1 pouce). La méthode couramment utilisée est une mesure de la résistance à l'ouverture à 180° d'un couple crochet/bande confort assemblé. Une bande de crochets de 13 mm de large et de 25,4 mm de long assemblée sur un support papier de 80g/m$^2$ de largeur 25,4 mm est pressée sur un échantillon de bande confort de dimension 50 mm x 50mm avec un rouleau simple de 2 kg, les orientations relatives des produits étant identiques à celles utilisées sur la couche-culotte. Une traction de 1 kg est ensuite réalisée pendant 10 secondes au support du crochet de manière à simuler la fermeture d'une couche-culotte, notamment d'une couche-culotte comportant des oreilles élastiques. Le papier supportant le crochet est ensuite inséré dans la mâchoire supérieure mobile d'un bâti de traction de type Synergie 200H de MTS System équipé d'une cellule dynamométrique de 100 N et la bande confort est insérée dans la mâchoire inférieure. La distance entre les deux mâchoires est de 50 mm. Pour mesurer la force d'ouverture, la partie haute du bâti effectue ensuite une translation de bas en haut à une vitesse constante de 305 mm/min. On relève alors la valeur de force maximale fournie par la machine ainsi qu'éventuellement la valeur d'énergie correspondant à l'aire sous la surface de la courbe prise sur les 13 premiers millimètres de course du bâti de traction.

Pour entraîner la désolidarisation d'une zone de fixation partielle suivant l'invention, la force nécessaire à cette désolidarisation est inférieure à la force normale définie ci-dessus.

En prévoyant ainsi deux types de fixation dans des zones différentes des régions de fixation entre l'élément support et les filaments formant les boucles, soit complète soit partielle, on augmente sensiblement l'efficacité d'accrochage de l'autoagrippant réalisé à partir d'un non-tissé comportant des boucles petites définies pour satisfaire l'aspect général (aspect visuel) du produit avant utilisation, mais trop petites pour obtenir les performances souhaitées en utilisant une bande confort avec uniquement des soudures complètes. On augmente aussi l'impression de solidité de l'accrochage ressentie par l'utilisateur. On réduit par ailleurs la formation de filaments/fibres libres formées par des boucles ouvertes.

De préférence, il est déposé sur l'élément support au moins une couche d'encre d'impression destinée à former un ou des motifs ou dessins.

Suivant un mode de réalisation préféré de l'invention, les fixations complète et partielle sont réalisées par soudure, notamment par calandrage.

Lorsque les fixations sont réalisées par calandrage, la tenue est assurée par une interdiffusion des molécules des fibres/filaments et du support. Le calandrage a pour effet de faire passer les fibres/filaments et le support intérieur entre deux cylindres, l'un d'entre eux au moins comportant une pluralité de protrusions et l'un d'entre eux au moins étant chauffé. Les protrusions permettent de presser les fibres/filaments les uns contre les autres et de créer les zones de fixation.

Lorsque la soudure est réalisée de manière complète, la pluralité de fibres/filaments est écrasée contre le support de sorte qu'on ne peut plus considérer chaque fibre/filament comme des éléments indépendants. Les fibres et filaments se soudent entre eux et au support.

De manière à caractériser la soudure différentielle du non-tissé, on peut définir le rapport de soudure suivant :

```
RS= (1-compacité de soudure partielle)/(1-compacité de
soudure complète)
```

La densité de vide (c'est-à-dire sans matière) présent dans la soudure partielle étant égale à 1 - compacité de la soudure partielle et La densité de vide présent dans la soudure complète étant égale à 1 - compacité de la soudure complète.

Préférentiellement, le rapport de soudure Rs est supérieur à 1,5, plus particulièrement supérieur à 2,5 et encore plus préférentiellement supérieur à 5, notamment 10, voire 100 ou plus.

En particulier, une fixation complète est une fixation telle que la matière obtenue au niveau de la zone de fixation a une fraction volumique ou compacité supérieure à 99%, tandis que la matière obtenue au niveau d'une zone de fixation partielle a une fraction volumique ou compacité inférieure à 99%.

La mesure de la fraction volumique peut se faire notamment par balayage (ou "scannage") 3D aux rayons X de la zone de fixation considérée. Pour la mesure, on prend comme zone de mesure (de préférence d'une dimension de 0,2 mm x 0,2 mm jusqu'à 0,5 mm x 0,5 mm) la zone de jonction entre les fibres/filaments et le support. On peut retirer 10%, de préférence 7,5%, encore plus préférablement 5% de part et d'autre de la zone considérée, pour ne pas avoir d'effet de bord.

Préférentiellement le test est réalisé au centre d'une zone de soudure homogène.

Ainsi, les filaments uniquement partiellement soudés ne sont pas totalement détruits par le calandrage et conservent une certaine intégrité dans les zones de fixation partielle, qui est telle que lorsqu'une traction est exercée sur le filament aux endroits où il n'a pas été soudé, la soudure partielle "lâche", augmentant ainsi l'ouverture utile du filament pour le crochet et augmentant ainsi la traction et notamment la sensation d'accroche. On entend par la soudure partielle "lâche" le fait qu'une partie au moins, de préférence la totalité des filaments de la soudure partielle en contact avec le support se désolidarise ou se détache du support.

Suivant un mode de réalisation préféré de l'invention, la pluralité de fibres et/ou filaments formant les boucles sont consolidés en au moins un non-tissé.

L' élément support est constitué d'au moins un non-tissé. Suivant un mode de réalisation préféré de l'invention, les fixations, notamment soudures, sont réalisées dans des régions en forme de bandes s'étendant dans la direction CD du stratifié, notamment en étant parallèles entre elles, les zones de fixation complètes et partielles étant réalisées en alternance le long d'une bande dans la direction CD.

Suivant un autre mode de réalisation, les zones de fixation complètes et partielles sont réalisées de manière à alterner à la fois dans la direction CD le long d'une bande donnée et dans la direction MD d'une bande à une bande voisine.

Suivant un autre mode de réalisation, les zones de fixation partielles et complètes sont réalisées alternées dans la direction MD d'une bande à une bande voisine. Suivant un autre mode de réalisation, les zones de fixation partielle sont réalisées en forme de demi-cercle, dont le diamètre est contigu à une zone de boucles.

De préférence, la nappe présente un différentiel de soudure supérieur à 1,5.

La présente invention vise aussi une couche-culotte comportant un ruban ou une nappe suivant l'invention, notamment la nappe ou le ruban forme la bande confort de la couche-culotte.

La présente invention vise également un système de fermeture auto-agrippante comportant d'une part un élément à crochets et d'autre part un élément à boucles, l'élément à boucles comportant un ruban ou une nappe suivant l'invention, en particulier un système de fermeture auto-agrippante pour une couche-culotte. L'élément à boucles formant la bande confort de la couche, tandis que l'élément à crochets formant une patte à crochets issue d'un bord de la couche, l'agencement étant tel que la coopération des crochets de l'élément à crochets dans les boucles de la bande confort assurant la fermeture et le maintien de la couche en position sur l'utilisateur.

Description succincte des dessins

Dans la suite, des modes de réalisation sont décrits uniquement à titre d'illustration en se reportant aux dessins dans lesquels :

- les figures 1A et 1B sont des schémas en coupe longitudinale d'un stratifié suivant l'invention illustrant le principe de l'invention, la figure 1A représentant l'état lorsqu'un crochet est introduit dans une boucle tandis que la figure 1B représente l'état une fois que le crochet a exercé une traction "normale" vers le haut ;

- la figure 1C est une vue de côté plus en détail du stratifié de la figure 1A, sans le crochet ;

- les figures 2A, 2B, 2C, 2D, 2E et 2F représentent de manière schématique des stratifiés suivant l'invention en perspective suivant divers modes de réalisation ;

- la figure 2G représente de manière schématique un stratifié suivant l'invention pour illustrer le concept de zone d'effet de bord des zones à soudure partielle, effet de bord qu'il convient notamment de prendre en compte lors de l'évaluation de la fraction volumique d'une zone de soudure partielle en retirant ces zones d'effet de bord de la zone de mesure ;

- la figure 2H représente de manière schématique un stratifié similaire à celui de la figure 2F dans lequel les zones de mesure de la fraction volumique ou compacité ont été représentées ;

- la figure 3A est une photographie vue de dessus au microscope (à agrandissement x 50) d'une zone circulaire d'un stratifié comportant une zone de soudure complète (au milieu) entre deux régions à boucles (à gauche et à droite à la figure) ;

- la figure 3B est une vue à une échelle quatre fois plus grande (à agrandissement x 200) de la partie encerclée de la zone de soudure complète de la figure 3A ;

- la figure 4A est une photographie vue de dessus schématique au microscope (à agrandissement x 50) d'une zone circulaire d'un stratifié comportant une zone de soudure partielle (au milieu) entre deux régions à boucles (à gauche et à droite à la figure) ;

- la figure 4B est une vue à une échelle quatre fois plus grande (à agrandissement x 200) de la partie encerclée de la zone de soudure partielle de la figure 4A ; et

- les figures 5 et 6 sont des vues sous forme schématique d'une section en coupe obtenue par logiciel lors de la mesure de la fraction volumique, respectivement pour les zones de soudure des figures 3B et 4B.

## Description détaillée de modes de réalisation

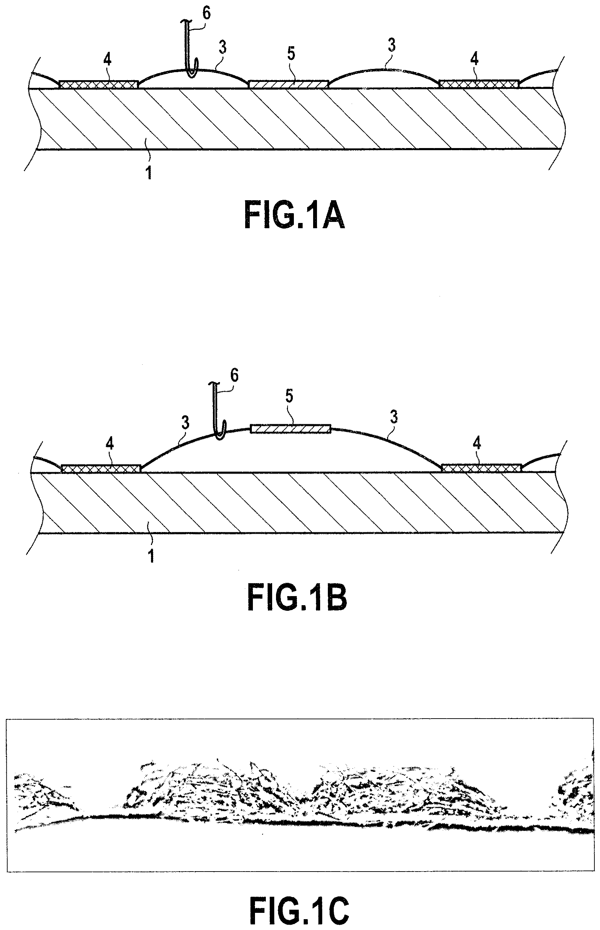

Aux figures 1A et 1B, il est représenté un stratifié suivant l'invention. Le stratifié comporte un élément de base sous la forme d'un non-tissé 1 ayant une face inférieure à la figure correspondant à la face intérieure destinée à se trouver du côté du corps de l'utilisateur de la couche-culotte dans le cas d'une application en tant que bande confort et qui comporte sur sa face opposée, supérieure à la figure et donc extérieure par rapport au corps de l'utilisateur de la couche-culotte, des motifs imprimés destinés à être vus à partir du côté extérieur et des filaments et/ou fibres 3 formant des boucles en étant fixés par soudure dans des régions 4, 5 de soudure au non-tissé 1.

Aux figures 1A et 1B, les filaments et/ou fibres 3 sont représentés schématiquement et forment en réalité un réseau de filaments et/ou de fibres comme représenté à la figure 1C.

Aux figures 1A et 1B, qui sont des vues schématiques en coupe, on peut voir que les régions de soudures comportent deux types de soudure différentes, à savoir des zones 4 de soudure complète, s'étendant dans la direction en longueur c'est-à-dire perpendiculairement à la figure et qui sont représentées par des traits hachurés en sombre, et des zones 5 de soudure partielle qui sont représentées en traits hachurés. Le stratifié réalise ainsi des boucles qui vont pouvoir coopérer avec des crochets pour réaliser une fixation par autoagrippant à crochets et boucles, notamment d'une couche-culotte, le stratifié étant disposé dans la zone centrale de la partie avant de la couche-culotte au niveau de la ceinture tandis que les crochets sont issus de pattes disposées aux extrémités latérales de la partie arrière de la couche-culotte également au niveau de la ceinture. Il est à noter cependant que sans sortir du cadre de l'invention, on pourrait prévoir que le stratifié à boucles soit disposé dans la partie arrière et que les crochets soient disposés dans la partie centrale de la ceinture de la partie avant de la couche-culotte.

On entend par boucles un filament et/ou une fibre qui comprend deux extrémités chacune solidaire du support en un point respectif du support ou en un même point du support. Une boucle peut également être formée de plusieurs filaments et/ou fibres solidarisés entre eux et dont au moins deux d'entre eux sont solidarisés au support, en un point ou en deux points respectifs distincts.

Les deux zones de soudure partielle et complète sont donc différentes et notamment la zone de soudure partielle peut se détacher du non-tissé 1 support sous l'action d'une force de traction d'un crochet 6 qui pénètre dans une boucle voisine d'une zone 5 de soudure partielle. On obtient ainsi la situation représentée à la figure 1B. Comme on le voit, les zones de soudure complète 4 n'ont pas été séparées du non-tissé 1 par l'action du crochet 6. En revanche, la zone de soudure partielle 5 s'est détachée du non-tissé 1 sous l'action du crochet 6 et on obtient ainsi maintenant une boucle de plus grande dimension constituée de deux filaments 3 et de la zone 5 qui comporte également les mêmes filaments en continuité.

Bien évidemment, cet effet est obtenu pour une force de traction dite normale du crochet. Si l'on applique une force de traction très élevée, l'agencement est tel que les filaments cassent avant les soudures complètes. Pour une traction normale correspondant à une traction exercée par une personne de force normale utilisant une couche-culotte de manière normale, la soudure 5 partielle va céder avant cassure des filaments 3 et ainsi réaliser une grande boucle.

En particulier, pour définir s'il s'agit d'une soudure partielle ou d'une soudure complète, on observe la fraction volumique de la matière contenue dans la zone de soudure que l'on veut évaluer. Une soudure complète est considérée comme une soudure dont les molécules des filaments et/ou fibres ont été complètement mélangées, de sorte à perdre leur intégrité dimensionnelle, ce qui se traduit par un passage, au niveau où la soudure est réalisée, d'un empilage de fibres dans le cas d'une soudure partielle à une masse de matière compacte dans le cas d'une soudure complète. Une zone à soudure partielle aura une grande porosité à l'air, à un liquide ou analogue et une fraction volumique ou compacité bien inférieure à 100% et notamment bien inférieure à celle d'une zone à soudure complète, dont la fraction volumique se rapproche de 100%.

Dans l'exemple des figures 1A et 1B, l'utilisateur fait pénétrer les crochets dans les boucles pour assurer la fermeture de la couche-culotte. Lors de l'ouverture, l'utilisateur tire la zone de crochets vers lui et exerce ainsi une force supérieure à la force de désolidarisation des soudures partielles du support. Ainsi, les boucles s'agrandissent et permettent d'obtenir une force d'ouverture normale supérieure à un stratifié similaire comprenant uniquement des soudures 3 et 4 complètes. Par exemple, on obtient ici une force d'ouverture mesurée à 4,5 N pour un stratifié à soudures complètes uniquement et 5,5 N pour le stratifié à double soudure complète et partielle.

En particulier, les soudures respectivement complète et partielle sont réalisées de manière différente et notamment avec une pression de calandrage différente. Pour ce faire, en particulier on peut soit réaliser des motifs sur les rouleaux de calandrage qui sont différents suivant que l'on veuille réaliser une zone de calandrage complet ou une zone de calandrage partiel. On peut également prévoir deux rouleaux de calandrage en succession, l'un pour réaliser un premier calandrage partiel avec une faible force de calandrage sur l'ensemble des zones de soudure puis ensuite un deuxième rouleau de calandrage pour terminer le calandrage complet dans les zones où l'on souhaite que le calandrage soit complet, tandis que les zones de calandrage partiel ne sont pas affectées par le deuxième rouleau de calandrage. Il est à noter que l'inverse de ces étapes est également possible et que les zones de calandrage de la première étape ne sont pas affectées par le deuxième rouleau de calandrage.

On peut également calandrer dans un premier temps le voile de fibres et/ou filaments et calandrer ensuite ce voile sur le support. Les zones préalablement calandrées du voile créent alors des zones de fixation partielle lorsqu'elles sont calandrées sur le support, la pression exercée sur la zone préalable de soudure lors de l'assemblage étant plus faible.

En particulier pour déterminer si une zone de calandrage est une zone de calandrage partiel ou une zone de calandrage complet, on réalise un balayage par rayon X de la zone de soudure, pour reconstituer sur la base de différentes coupes transversales le volume de matière contenu.

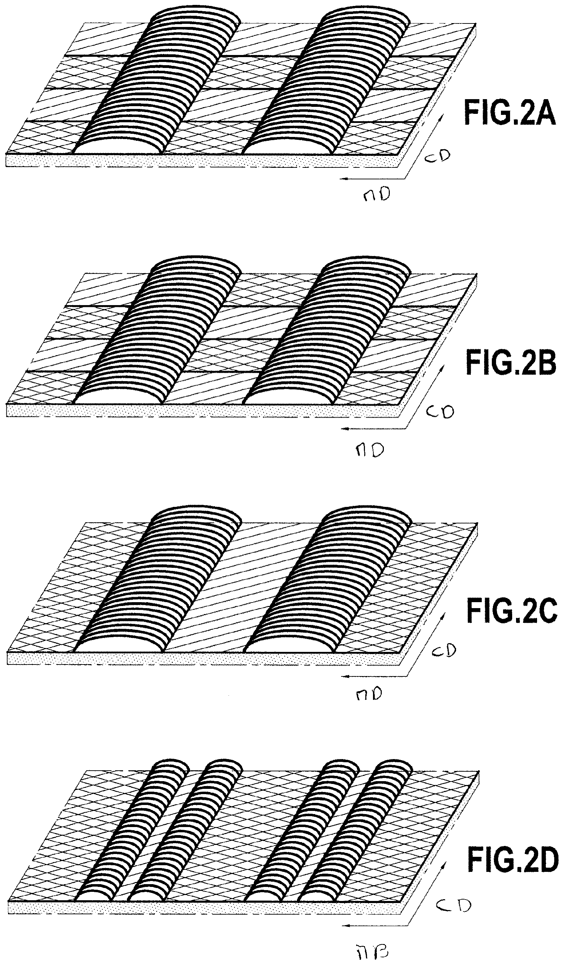

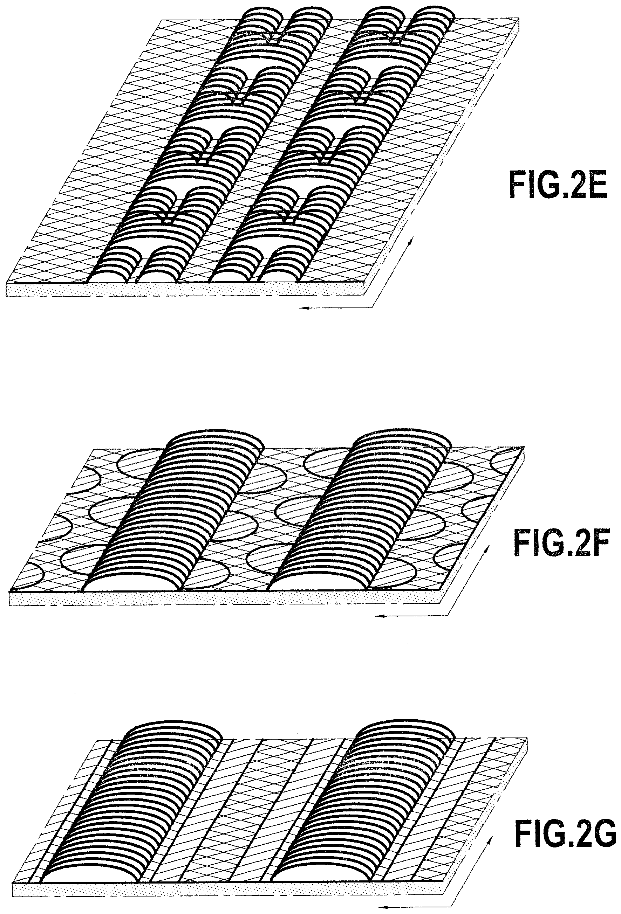

Aux figures 2A, 2B, 2C, 2D, 2E, 2F et 2G, il est représenté divers modes de réalisation possibles pour réaliser le stratifié à boucles avec des soudures différentielles suivant le principe de l'invention.

Ainsi, à la figure 2A, les régions de soudure forment des bandes s'étendant dans la direction CD en alternance avec des zones dans lesquelles les filaments ne sont pas soudés pour former des boucles par rapport à l'élément 1 support. En outre, comme représenté aux figures, dans chaque région de fixation par soudure en forme de bande des zones 4 de soudure complète alternent avec des zones 5 de soudure partielle dans la direction CD. Comme représenté, chaque zone a sensiblement la même extension en longueur dans la direction CD, mais d'autres configurations pourraient être envisagées, par exemple des zones partielles en forme de disque circulaire ou de tout autre forme, identiques les uns aux autres ou non, à distance les uns des autres en étant entouré par une zone de soudure complète, ou à distance de la zone de soudure complète et isolée de celle-ci.

Suivant un autre mode de réalisation représenté à la figure 2B, l'alternance dans la direction CD est complétée par une alternance dans la direction MD, pour ainsi former entre les zones de soudures partielle et complète une sorte de damier.

Suivant un autre mode de réalisation représenté à la figure 2C, les bandes de soudures partielles et complètes sont continues dans la direction CD mais alternent les unes avec les autres dans la direction MD.

A la figure 2D est représenté encore un autre mode de réalisation dans lequel des bandes de soudure complètes ont une plus grande largeur que des bandes de soudure partielle formées alternativement avec les bandes de soudure complètes, les bandes de soudure s'étendant dans la direction CD. En particulier, les bandes de soudure partielle sont minces, notamment au point d'avoir sensiblement la forme d'une simple ligne de soudure.

A la figure 2E, il est représenté un mode de réalisation sensiblement identique à celui de la figure 2D, la seule différence étant que les lignes de soudure partielle sont en outre discontinues dans la direction CD.

A la figure 2F, il est représenté un mode de réalisation dans lequel les zones de soudure partielle sont réalisées sous la forme de rangées de zones en demi cercle espacées les unes des autres dans la direction CD, les rangées de demi cercles étant en outre de préférence décalées les unes par rapport aux autres d'une rangée à l'autre, par exemple d'une demi distance entre deux demi cercles successifs d'une même rangée. En outre la ligne formant le diamètre du demi cercle d'une rangée se trouve du côté des zones à boucles. En particulier, comme représentée à la figure, les concavités des demi cercles sont orientées dans des directions opposées d'une rangée à la rangée suivante.

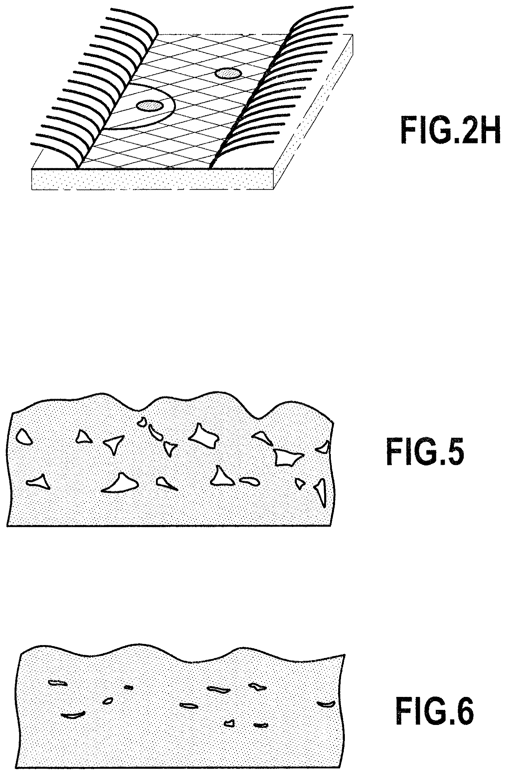

A la figure 2H, on a représenté un mode de réalisation similaire à celui de la figure 2F dans lequel les zones de mesure de la fraction volumique ou compacité ont été représentées. Les zones de mesures sont indiquées par des forme sensiblement circulaire en hachuré foncé, respectivement dans la zone de soudure partielle en forme de demi disque et dans la zone de soudure complète.

A la figure 2G, il est représenté un mode de réalisation dans lequel deux rangées de boucles sont séparées par une zone de soudure ; La zone de soudure comporte une région en forme de bande centrale de soudure complète (s'étendant en longueur dans la direction CD), entre deux zones latérales de soudure partielle, séparant chacune la bande centrale de soudure complète d'une rangée de boucles respective. Dans chacune des zones latérales de soudure partielle, on définit en outre une bande dite d'effet de bord dans laquelle la soudure n'est ni complète ni vraiment partielle et correspond à des effets de bord apparaissant lors des calandrages respectivement de la zone complète et des zones partielles. Au-delà de ces zones d'effet de bord, et jusqu'à la rangée de boucle contiguë, s'étend la zone de soudure partielle.

Lors de la mesure de la fraction volumique, on peut, pour les zones à soudure partielle, prendre en compte ces zones d'effet de bord en les ôtant de la zone mesurée. On peut faire ainsi dans l'ensemble des modes de réalisations ici décrit, et également de manière générale pour tout autre mode de réalisation analogue.

Dans les différents modes de réalisation représentés ci-dessus, on a représenté des fibres et/ou filaments (ces deux termes sont utilisés dans la présente demande de manière équivalente et interchangeable) qui ne sont pas consolidés les uns avec les autres. Cependant, on pourrait également prévoir d'utiliser des filaments consolidés ensemble en un non-tissé et réaliser les soudures, respectivement partielle et complète sur ce non-tissé.

Dans la présente demande, on entend par non-tissé un produit obtenu à l'issue de la formation d'une nappe de fibres et/ou de filaments qui ont été consolidés. La consolidation peut être mécanique, chimique ou thermique et se traduit par la présence de liaison entre les fibres et/ou les filaments. Cette consolidation peut être directe, c'est-à-dire faite directement entre les fibres et/ou filaments par soudure, ou elle peut être indirecte, c'est-à-dire par l'intermédiaire d'une couche intermédiaire entre les fibres et/ou les filaments, par exemple une couche de colle ou une couche de liant. Le terme non-tissé se rapporte à une structure en forme de nappe ou de ruban de fibres et/ou filaments qui sont entrelacées d'une manière non uniforme, irrégulière ou au hasard. Un non-tissé peut avoir une structure de couche unique ou une structure à couches multiples. Un non-tissé peut également être réuni à un autre matériau tel qu'un film pour former un stratifié. Un non-tissé peut être réalisé à partir de différents matériaux synthétiques et/ou naturels. Les matériaux naturels à titre d'exemple sont des fibres de cellulose, telles que coton, jute ou pâte à papier et analogue et peuvent également inclure des fibres de cellulose re-traitées, telles que la rayonne ou la viscose. Les fibres naturelles pour un matériau non-tissé peuvent être préparées en utilisant divers procédés tels que le cardage. Des matériaux synthétiques à titre d'exemple comportent, mais sans s'y limiter, des polymères thermoplastiques synthétiques, qui sont connus pour former des fibres qui incluent, sans s'y limiter, les polyoléfines, par exemple le polyéthylène, polypropylène, polybutylène et analogue ; le polyamide, par exemple le polyamide 6, polyamide 6.6, polyamide 10, polyamide 12 et analogue ; des polyesters, par exemple des polyéthylènes téréphtalates, des polybutylènes téréphtalates, des acides polylactiques et analogues, des polycarbonates, des polystyrènes, des élastomères thermoplastiques, des vinyles polymères, des polyuréthanes et des mélanges et des copolymères de ces derniers.

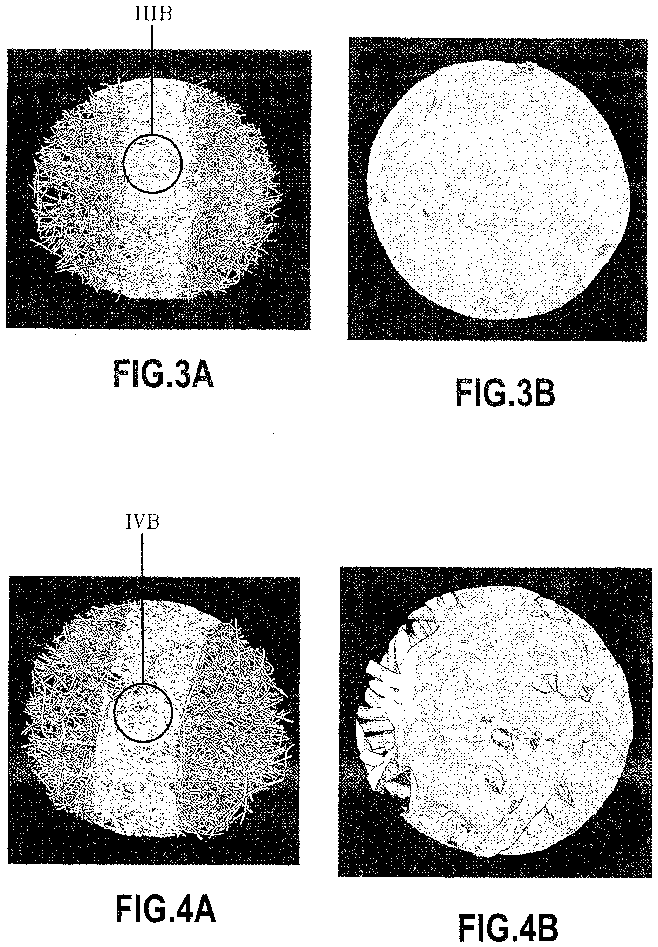

Aux figures 3A, 3B, 4A, 4B, 5 et 6, il est représenté en vue de dessus (3A, 3B, 4A, 4B) ou en coupe transversale (5, 6), respectivement sous la forme de photographies et de schémas des parties d'un non-tissé à boucles dans lequel il a été formé des zones à calandrage complet (Figures 3A, 3B et 6) et des zones à calandrage partiel (Figures 4A, 4B et 5).

Dans les zones à calandrage complet, la zone délimitée par le cercle C à la figure 3A et représenté à la figure 3B, la majorité des fibres et/ou filaments ont été écrasé et ne sont pas discernables les uns des autres, de sorte que la zone est principalement recouverte d'un champ opaque. Dans cette zone les fibres ont été fortement aplaties. Comme on peut le voir en coupe transversale à la figure 5, la fraction volumique est élevée, très peu d'espace vide subsistant dans la matière des fibres soudées ensemble.

Dans les zones à calandrage partiel, notamment la zone délimitée par le cercle B à la figure 4A et représenté à la figure 4B, la majorité des fibres et/ou filaments n'ont été que peu écrasé et sont discernables les unes des autres. Comme on peut le voir en coupe transversale à la figure 6, la fraction volumique est plus petite qu'à la figure 5, un espace vide (c'est-à-dire sans matière) non négligeable subsistant dans la matière des fibres soudées ensemble.

On pourrait utiliser des matières différentes pour les fibres et/ou filaments destinés à être soudés ou fixés de manière complète et pour les fibres et/ou filaments destinés à être soudés ou fixés de manière partielle.

Pour un stratifié, la direction MD (Machine Direction ou direction machine) est la direction dans laquelle le stratifié est déroulé lors de sa fabrication et la direction CD (Cross Direction ou direction transversale) est la direction perpendiculaire à la direction MD, dans le plan du stratifié.

Selon une variante de réalisation de l'invention, on pourrait envisager plus de deux types de soudure, par exemple trois types de soudure.

Selon des variantes de réalisation, on pourra envisager différentes soudures partielles en faisant varier leurs dimensions, leur forme, leur fraction volumique, permettant ainsi d'obtenir les performances requises pour chaque type d'application.

Un produit avec une alternance de soudures partielles et de soudures complètes en CD du type de la figure 2A permet d'obtenir une augmentation d'environ 10% des performances en pelage par rapport à l'art antérieur. On obtient ainsi une force moyenne de 4,1N en pelage contre 3,7 N pour un produit réalisé uniquement avec des soudures complètes.

On entend par non-tissé à boucles, un non-tissé formant des boucles au moins après sa liaison au support.

De manière générale, les fibres et les filaments diffèrent principalement par leur longueur et par leur procédé de fabrication.

On entend par filaments les éléments unitaires, de très grandes longueurs vis à vis du diamètre dans lequel s'inscrit leur section, extrudés de manière continue pour former directement une nappe de non-tissé qui peut être ensuite consolidée par thermoliage ou tout autre moyen pour permettre l'atteinte des performances souhaitées et/ou leur transport. De préférence, les filaments présentent une longueur supérieure à 120 mm.

On entend par fibre le terme générique pour désigner une matière textile ou un élément de matière textile de longueur réduite, inférieure à la longueur des filaments, et susceptible d'être filée et/ou utilisée dans la réalisation de non-tissés. On distingue deux types de fibres, les fibres courtes formées de matière discontinue de faible longueur inférieure à 70mm (préférentiellement de 25 mm à 60 mm) et les fibres longues formées de manière discontinue de grande longueur supérieure à 70 mm (préférentiellement de 80 mm à 120 mm).

A la différence des filaments qui sont consolidés directement après avoir été extrudés, les fibres sont communément orientées et organisées en nappe lors d'une étape de cardage bien connue de l'homme du métier. Cette nappe peut être ensuite consolidée par thermoliage ou tout autre moyen pour permettre l'atteinte des performances souhaitées et/ou leur transport.

## Revendications

1. Nappe à boucles en forme de stratifié comportant un élément support (1) intérieur constitué d'un non-tissé et une pluralité de fibres et/ou filaments (3) fixés sur la face extérieure de l'élément support de manière à former des boucles pour une coopération avec des crochets, la fixation étant réalisée dans au moins une région de fixation tandis que dans au moins une autre région dite bouclée les filaments et/ou fibres ne sont pas fixés à l'élément support pour ainsi former des boucles, **caractérisé en ce que**, dans au moins une zone (5) de la au moins une région de fixation, la fixation n'est réalisée que de manière partielle tandis que dans au moins une autre zone (4) de la au moins une région de fixation la fixation est réalisée de manière complète.

2. Nappe suivant la revendication 1, **caractérisée en ce que** les fixations complète et partielle sont réalisées par soudure, notamment par calandrage.

3. Nappe suivant la revendication 1 ou 2, **caractérisée en ce que** la pluralité de fibres et/ou filaments (3) formant les boucles (7) sont consolidés en au moins un non-tissé.

4. Nappe suivant l'une des revendications 1 à 3, **caractérisée en ce qu'**il est déposé sur l'élément support (1) au moins une couche d'encre d'impression destinée à former un ou des motifs ou dessins.

5. Nappe suivant l'une des revendications 1 à 4, **caractérisée en ce que** les fixations, notamment soudures, sont réalisées dans des régions en forme de bandes s'étendant dans la direction CD du stratifié, notamment en étant parallèles entre elles, les zones de fixation complète et partielle étant réalisées en alternance le long d'une bande dans la direction CD.

6. Nappe suivant l'une des revendications 1 à 5, **caractérisée en ce que** les zones de fixation complète et partielle sont réalisées de manière à alterner à la fois dans la direction CD le long d'une bande donnée et dans la direction MD d'une bande à une bande voisine.

7. Nappe suivant l'une des revendications 1 à 6, **caractérisée en ce que** les zones de fixation partielle et complète sont réalisées alternées dans la direction MD d'une bande à une bande voisine.

8. Nappe suivant l'une des revendications précédentes, **caractérisée en ce que** les zones de fixation partielle sont réalisées en forme de demi-cercle, dont le diamètre est contigu à une zone de boucles.

9. Nappe suivant l'une des revendications précédentes, **caractérisée en ce qu'**elle présente un différentiel de soudure supérieur à 1,5.

10. Couche-culotte comportant une nappe suivant l'une des revendications précédentes.

11. Couche-culotte suivant la revendication 10, **caractérisée en ce que** la nappe forme la bande confort de la couche-culotte.

12. Système de fermeture auto-agrippante comportant d'une part un élément à boucle comportant une nappe suivant l'une des revendications 1 à 9 et d'autre part un élément à crochets dont les crochets sont agencés pour pouvoir s'accrocher dans les boucles pour réaliser la fermeture.


**Patentansprüche**

1. Lage mit Schlaufen in Form eines Schichtstoffs, der ein aus einem Vlies bestehendes inneres Trägerelement (1) und eine Vielzahl von Fasern und/oder Filamenten (3) aufweist, die an der Außenseite des Trägerelements so befestigt sind, dass sie Schlaufen für eine Zusammenwirkung mit Haken bilden, wobei die Befestigung in mindestens einem Befestigungsbereich durchgeführt wird, während in mindestens einem anderen, so genannten geschlauften Bereich die Filamente und/oder Fasern nicht am Trägerelement befestigt sind, um so Schlaufen zu bilden, **dadurch gekennzeichnet, dass** in mindestens einer Zone (5) des mindestens einen Befestigungsbereichs die Befestigung nur teilweise durchgeführt wird, während in mindestens einer anderen Zone (4) des mindestens einen Befestigungsbereichs die Befestigung vollständig durchgeführt wird.

2. Lage nach Anspruch 1, **dadurch gekennzeichnet, dass** die vollständige und die teilweise Befestigung durch Schweißen, insbesondere Kalandrieren erfolgt.

3. Lage nach Anspruch 1 oder 2, **dadurch gekennzeichnet, dass** die Vielzahl von die Schlaufen (7) bildenden Fasern und/oder Filamenten (3) in mindestens einem Vlies verfestigt sind.

4. Lage nach einem der Ansprüche 1 bis 3, **dadurch gekennzeichnet, dass** auf das Trägerelement (1) mindestens eine Druckfarbenschicht aufgebracht wird, die dazu bestimmt ist, ein oder mehrere Muster oder Zeichnungen zu bilden.

5. Lage nach einem der Ansprüche 1 bis 4, **dadurch gekennzeichnet, dass** die Befestigungen, insbesondere Schweißungen, in streifenförmigen Bereichen durchgeführt werden, die sich in der Richtung CD des Schichtstoffs erstrecken, indem sie insbesondere parallel zueinander sind, wobei die Zonen vollständiger und teilweiser Befestigung abwechselnd entlang eines Streifens in der Richtung CD hergestellt werden.

6. Lage nach einem der Ansprüche 1 bis 5, **dadurch gekennzeichnet, dass** die Zonen vollständiger und teilweise Befestigung so hergestellt werden, dass sie sich in der Richtung CD entlang eines gegebenen Streifens und in der Richtung MD von einem Streifen zu einem benachbarten Streifen abwechseln.

7. Lage nach einem der Ansprüche 1 bis 6, **dadurch gekennzeichnet, dass** die Zonen teilweiser und vollständiger Befestigung abwechselnd in der Richtung MD von einem Streifen zu einem benachbarten Streifen hergestellt werden.

8. Lage nach einem der vorhergehenden Ansprüche, **dadurch gekennzeichnet, dass** die Zonen teilweiser Befestigung in Form eines Halbkreises hergestellt werden, dessen Durchmesser an eine Zone von Schlaufen angrenzt.

9. Lage nach einem der vorhergehenden Ansprüche, **dadurch gekennzeichnet, dass** sie eine differentielle Schweißung von mehr als 1,5 aufweist.

10. Höschenwindel, die eine Lage nach einem der vorhergehenden Ansprüche aufweist.

11. Höschenwindel nach Anspruch 10, **dadurch gekennzeichnet, dass** die Lage das Komfortstreifen der Höschenwindel bildet.

12. Klettverschlusssystem, das einerseits ein Schlaufenelement, das eine Lage nach einem der Ansprüche 1 bis 9 aufweist, und andererseits ein Element mit Haken aufweist, dessen Haken eingerichtet sind, um sich in die Schlaufen einhaken zu können, um das Schließen durchzuführen.

**Claims**

1. A looped layer in the form of a laminate comprising an internal support element (1), made of a non-woven fabric, and a plurality of fibers and/or filaments (3) secured to the external face of the support element so as to form loops for engaging with hooks, the attachment being made in at least one attachment region, while in at least one other so-called looped region the filaments and/or fibers are not secured to the support element so as to thus form loops, **characterized in that** in at least one zone (5) of the at least one attachment region the attachment is only carried out in a partial manner, while in at least one other zone (4) of the at least one attachment region the attachment is carried out in a complete manner.

2. The layer according to Claim 1, **characterized in that** the complete and partial attachments are achieved by welding, in particular by calendering.

3. The layer according to Claim 1 or 2, **characterized in that** the plurality of fibers and/or filaments (3) forming the loops (7) are consolidated into at least one non-woven fabric.

4. The layer according to any of Claims 1 to 3, **characterized in that** at least one layer of printing ink intended to form a pattern or patterns or designs is deposited onto the support element (1).

5. The layer according to any of Claims 1 to 4, **characterized in that** the attachments, in particular welds, are made in regions in the form of strips extending in the CD direction of the laminate, in particular parallel to one another, the complete and partial attachment zones being produced alternately along a strip in the CD direction.

6. The layer according to any of Claims 1 to 5, **characterized in that** the complete and partial attachment zones are produced so as to alternate both in the CD direction along a given strip and in the MD direction of a strip to an adjacent strip.

7. The layer according to any of Claims 1 to 6, **characterized in that** the partial and complete attachment zones are made alternately in the MD direction of a strip to an adjacent strip.

8. The layer according to any of the preceding claims, **characterized in that** the partial attachment zones are made in the form of a semi-circle of which the diameter is contiguous to a loop zone.

9. The layer according to any of the preceding claims, **characterized in that** it exhibits a weld differential greater than 1.5.

10. A diaper comprising a layer according to any of the preceding claims.

11. A diaper according to Claim 10, **characterized in that** the layer forms the landing zone of the diaper.

12. A self-fastening closure system comprising on the one hand a loop element comprising a layer according to any of Claims 1 to 9 and on the other hand a hook element, the hooks of which are arranged to be able to engage in the loops in order to provide the closure.

**FIG.1A**

**FIG.1B**

**FIG.1C**

FIG.2A

FIG.2B

FIG.2C

FIG.2D

FIG.2E

FIG.2F

FIG.2G

FIG.2H

FIG.5

FIG.6

FIG.3A

FIG.3B

FIG.4A

FIG.4B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20040178544 A
- EP 0800808 A